# FASCICULE DE BREVET EUROPEEN

(11) **EP 2 097 524 B1**
(45) Date de publication et mention de la délivrance du brevet: **13.04.2016**
(21) Numéro de dépôt: 07872389.7
(22) Date de dépôt: 18.12.2007
(51) Int. Cl.: C12N 15/10

(54) **PROCEDE DE DIVERSIFICATION ALEATOIRE D'UNE SEQUENCE GENETIQUE PERMETTANT DE PRESERVER L'IDENTITE DE CERTAINS SEGMENTS INTERNES DE LADITE SEQUENCE GENETIQUE**
VERFAHREN FÜR DIE ZUFÄLLIGE DIVERSIFIZIERUNG EINER GENETISCHEN SEQUENZ UNTER BEWAHRUNG DER IDENTITÄT EINIGER INNENSEGMENTE DIESER GENETISCHEN SEQUENZ
METHOD FOR THE RANDOM DIVERSIFICATION OF A GENETIC SEQUENCE WHILE PRESERVING THE IDENTITY OF SOME INNER SEGMENTS OF SAID GENETIC SEQUENCE

(30) Priorité: 20.12.2006 FR 0655723
(43) Date de publication de la demande: 09.09.2009
(73) Titulaire: Pherecydes Pharma, 75008 Paris (FR)
(72) Inventeur: IRIS, François, 92370 Chaville (FR)
(74) Mandataire: Santarelli
(86) Numéro de dépôt international: PCT/FR2007/002102
(87) Numéro de publication internationale: WO 2008/093010

(56) Documents cités:
- EP-A- 1 696 037
- WO-A-98/16661
- WO-A-99/23107
- WO-A-2006/066224
- WO-A2-02/07742
- YOICHI M ET AL: "Alteration of tail fiber protein gp38 enables T2 phage to infect Escherichia coli O157:H7" JOURNAL OF BIOTECHNOLOGY, ELSEVIER SCIENCE PUBLISHERS, AMSTERDAM, NL, vol. 115, no. 1, 12 janvier 2005 (2005-01-12), pages 101-107, XP004966993 ISSN: 0168-1656
- PATTEN P A ET AL: "APPLICATIONS OF DNA SHUFFLING TO PHARMACEUTICALS AND VACCINES" CURRENT OPINION IN BIOTECHNOLOGY, LONDON, GB, vol. 8, 1997, pages 724-733, XP002916609 ISSN: 0958-1669
- KREBS B ET AL: "High-throughput generation and engineering of recombinant human antibodies", JOURNAL OF IMMUNOLOGICAL METHODS, ELSEVIER SCIENCE PUBLISHERS B.V.,AMSTERDAM, NL, vol. 254, no. 1-2, 1 August 2001 (2001-08-01), pages 67-84, XP004245443, ISSN: 0022-1759, DOI: 10.1016/S0022-1759(01)00398-2

## Description

La présente invention vise un procédé très général pour diversifier de manière aléatoire une séquence nucléotidique S par PCR, tout en préservant l'identité de certains domaines ou segments de cette séquence, et décrit une banque de séquences nucléotidiques ainsi diversifiées, et plus particulièrement une banque de séquences codant des protéines de ciblage de bactériophages modifiées.

Les bactériophages se présentent comme des virus capables d'infecter spécifiquement les bactéries et de s'y répliquer. Leur existence a été mise en évidence au début du XX^{eme} siècle par le Britannique Frederick Twort et le Québécois Félix d'Hérelle.

Les bactériophages occupent toutes les niches écologiques où se trouvent des bactéries. Ils se manifestent principalement sous deux formes: la forme lysogénique, par laquelle ils peuvent demeurer de manière quiescente à l'intérieur de leur hôte, ou bien sous forme lytique, lorsqu'ils se répliquent de manière active en lysant la cellule bactérienne. La forme lytique amène les bactériophages à être libéré en grand nombre dans l'environnement sous une forme infectieuse.

Afin de conserver leur caractère infectieux vis-à-vis de leurs hôtes qui connaissent parfois des mutations rapides, les bactériophages sont amenés à évoluer constamment. De ce fait, Ils présentent naturellement un degré élevé de spécialisation pour les espèces bactériennes qu'ils parasitent et sont très diversifiés.

Dès leur découverte, les bactériophages ont été considérés comme un moyen de lutter contre les infections bactériennes, et ce bien avant l'ère des antibiotiques.

La démarche consistant à identifier dans la nature des bactériophages spécifique d'une bactérie pathogène afin de traiter les patients infectés par cette bactérie s'est ainsi développée en Russie et dans les pays de l'ancien bloc soviétique pendant la première moitié du XXeme siècle.

Cependant l'emploi des antibiotiques, dont le spectre est généralement beaucoup plus large, s'est généralisé dans la seconde moitié du XXeme siècle de façon massive, sans exploiter toutes les possibilités offertes par les bactériophages.

Aujourd'hui, face à l'apparition de souches bactériennes multi-résistantes aux antibiotiques, et au vu des difficultés rencontrées pas la communauté scientifique pour mettre au point de nouveaux antibiotiques, les bactériophages suscitent un nouvel intérêt pour le traitement des infections bactériennes difficiles à éradiquer, notamment en cas de contaminations nosocomiales [Thiel, K., Nature Biotechnology, 2004, 22 :31-36].

Certaines difficultés persistent toutefois dans l'utilisation des bactériophages, notamment dans le fait que les bactéries peuvent échapper aux bactériophages en masquant ou en modifiant les éléments constitutifs de leur paroi externe.

Le cycle de réplication des bactériophages nécessite, en effet, une étape de reconnaissance et d'adhésion du bactériophage à la paroi de la bactérie hôte, laquelle conditionne la possibilité pour le bactériophage d'infecter la bactérie, c'est-à-dire d'injecter le matériel génétique contenu dans sa capside à l'intérieur du cytoplasme de la bactérie.

Le bactériophage T4, par exemple, est un bactériophage qui infecte les bactéries de type *Escherichia coli*, dont le cycle de réplication dure environ 30 minutes à 37 °C. Ce cycle de réplication débute immédiatement après la reconnaissance de la bactérie hôte par le bactériophage, par la phase d'absorption et de pénétration. Il se traduit par l'arrêt immédiat de l'expression des gènes de la bactérie hôte, la synthèse des enzymes nécessaires à la réplication du phage, 5 minutes après l'infection, puis la réplication de l'ADN (démarrant après 10 minutes) et la formation du virus (démarrant après 12 minutes). Le cycle de réplication conduit à l'éclatement de la bactérie (après 30 minutes) et la libération dans l'environnement d'environ cinquante bactériophages par bactérie lysée.

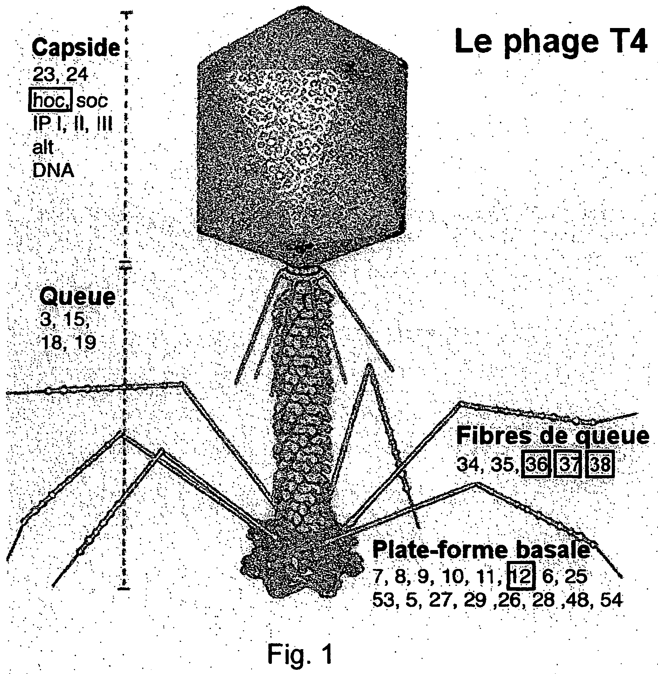

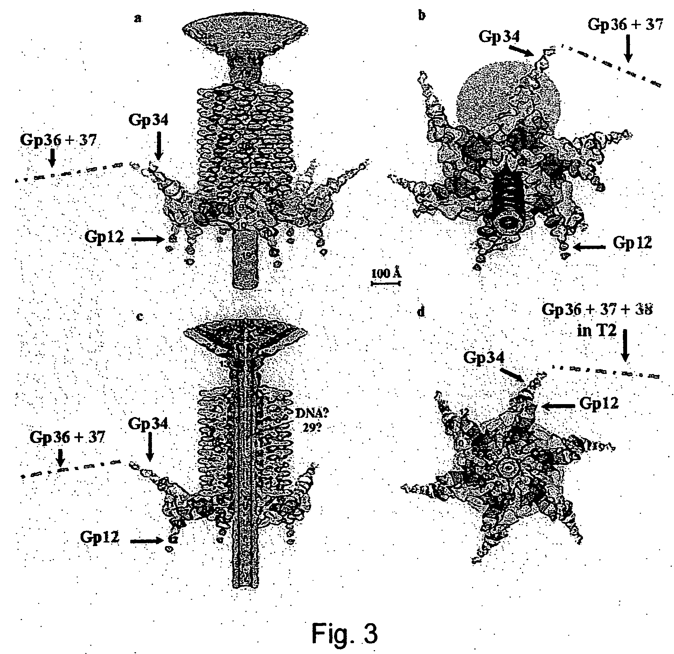

L'adhésion à la bactérie est essentiellement assurée par les protéines de la plate-forme basale (baseplate) servant d'ancrage au bactériophage et la reconnaissance est assurée plus particulièrement par des protéines formant les filaments périphériques, appelées « fibres de queue ». Néanmoins, les protéines de fibres de queue et du baseplate peuvent être à la fois impliquées dans la reconnaissance et l'adhésion du bactériophage à la paroi bactérienne. L'ensemble de ces protéines dites de « ciblage » est représenté dans les figures 1 et 3 de la présente demande.

Parmi les protéines impliquées dans cette reconnaissance ou cette adhésion chez le phage T4, on peut citer plus particulièrement les glycoprotéines GP12 de la plate forme basale, et les glycoprotéines GP36, GP37 et GP38 des fibres de queue.

Afin de limiter l'émergence de bactéries résistantes au dispositif de reconnaissance des bactériophages, il est généralement proposé d'utiliser simultanément différents formes de bactériophages capables de cibler une même bactérie.

Ces bactériophages sont prélevés dans la nature ou issus de collections, pour former ensemble ce qu'on appelle « un cocktail de bactériophages ».

Cependant, il convient pour la mise au point de ces cocktails de bactériophages de sélectionner individuellement et rigoureusement les bactériophages qui les composent, en s'assurant, notamment, que ces bactériophages sont lytiques et non lysogèniques ou partiellement lysogèniques, comme c'est le cas souvent des bactériophages prélevés dans le milieu naturel.

La nécessité de tester individuellement les bactériophages pour s'assurer de leur efficacité réelle rend le développement des cocktails de bactériophages long et fastidieux, et ce d'autant qu'il faut prévoir un cocktail différent pour chaque bactérie considérée.

La demande WO 01/51066 décrit une telle préparation de bactériophages comprenant six bactériophages différents utilisée en tant qu'agent de conservation de produits alimentaires frais pour détruire la bactérie *Listeria monocytogenes,* responsable de la listériose. Cette préparation naturelle est conditionnée en pulvérisateur pour être pulvérisée sur de la viande ou des produits laitiers. Elle est sans danger pour l'homme, les animaux ou les plantes, car les bactériophages ne peuvent infecter que des bactéries du genre Listeria et non les cellules des organismes pluricellulaires.

Pour remédier aux problèmes posés par la sélection des bactériophages naturels, il est proposé dans la demande WO 06/066224 une méthode pour obtenir des bactériophages dont les protéines de ciblage sont modifiées en vue de cibler spécifiquement un facteur de virulence donné. Les facteurs de virulence sont des molécules décrites comme étant nécessaires à la bactérie pour développer une infection. Ces molécules sont considérées comme des éléments stables, moins susceptibles de varier au cours de l'infection que les éléments de structures externes comme, par exemple, les lipopolysaccharides. Plus particulièrement, cette méthode propose de sélectionner une protéine issue d'un bactériophage naturel (par exemple GP37 du phage T4) capable de reconnaître un facteur de virulence décrit dans la littérature (par exemple OmpC d'E.coli), de transférer le gène codant cette protéine dans un bactériophage lambda et d'utiliser le bactériophage lambda pour modifier cette protéine. La modification de la protéine consiste à opérer des échanges entre les différents domaines impliqués dans la reconnaissance du facteur de virulence (par exemples les domaines His de GP37). Les phages lambda sont ensuite testés pour leur capacité à adhérer au facteur de virulence ciblé. Cette méthode, qui s'apparente à la technique du phage display, permet d'isoler différents variants du bactériophage lambda capables de cibler le facteur de virulence, et ainsi de disposer de différentes protéines de ciblage. Les gènes correspondants à ces différentes protéines de ciblage, peuvent, par la suite, être transférés dans des bactériophages infectieux. Ces bactériophages peuvent alors constituer des cocktails de phages actifs vis-à-vis de la bactérie porteuse du facteur de virulence ciblé au départ.

Cette méthode constitue un progrès dans l'obtention de bactériophages diversifiés pour la mise au point de cocktails de bactériophages. Néanmoins, il reste que de tels cocktails ne visent que l'espèce bactérienne exprimant le facteur de virulence ciblé initialement.

Cette méthode ne peut donc être mise en oeuvre que pour des bactéries pathogènes cultivables sur lesquelles une identification préalable des facteurs de virulence a pu être effectuée.

Pour rendre l'emploi des bactériophages plus universel, il serait utile de disposer de bactériophages infectieux vis-à-vis d'un plus grand nombre d'espèces, par exemple, en créant des bactériophages dont le spectre d'infectivité serait modifié ou élargi. De tels bactériophages pourraient être utilisés à l'encontre de nouvelles espèces bactériennes, notamment à l'encontre de bactéries pathogènes émergentes ou à l'origine d'infections nosocomiales.

Néanmoins, la production de phages modifiés dans leur spectre d'infectivité se heurte à la contrainte technique que l'obtention des bactériophages est dépendante de l'hôte bactérien dans lequel le bactériophage est transformé puis multiplié. Les étapes de modification génétique des phages comprennent généralement plusieurs étapes de réplication dans un seul hôte bactérien.

Dans les expériences décrites dans les documents de l'art antérieur précités, de nombreux cycles de réplication sont nécessaires pour modifier les bactériophages servant à cibler les facteurs de virulence. La recombinaison homologue qui est la technique la plus souvent rencontrée pour transformer le génome des bactériophages implique de nombreux cycles de réplication et de sélection successifs chez la bactérie. Or, les bactériophages modifiés, s'ils parviennent à acquérir la capacité d'infecter des hôtes différents de leurs hôtes habituels, peuvent aussi perdre la capacité d'infecter l'hôte utilisé pour leur réplication. Dès lors, ils se trouvent éliminés de la sélection et échappent à l'expérimentateur. Il en résulte une importante perte de diversité des phages modifiés susceptibles d'être obtenus.

Pour remédier aux difficultés précitées, la présente invention propose un nouveau procédé consistant à diversifier de manière aléatoire les séquences nucléotidiques, notamment celles codant les protéines de ciblage des bactériophages, par insertion dans leurs gènes de séquences d'ADN produites de manière aléatoire.

Ce procédé est particulièrement utile pour produire des copies de gènes comprenant des segments mutés de manière aléatoire, notamment en vue de leur clonage dans des vecteurs d'expression ou de recombinaison homologue.

Le présent procédé selon l'invention a été mis au point initialement pour diversifier les protéines de ciblage des bactériophages et ainsi obtenir par recombinaison homologue des bactériophages recombinants dont la spécificité d'hôte varie.

Cependant, il ne saurait se limiter au seul domaine des bactériophages car il peut être appliqué à toute séquence d'ADN ou d'ARN.

Les domaines d'applications sont nombreux notamment dans le domaine médical, dès lors que l'on cherche à modifier une séquence génétique de manière aléatoire dans ses domaines variables, tout en préservant l'identité des domaines constants, qui sont généralement essentiels à la fonctionnalité de la protéine.

Le procédé selon l'invention est détaillé ci-après, ainsi que son application plus particulière dans le domaine des bactériophages.
**Figure 1** : représentation du bactériophage T4 montrant les différentes éléments constitutifs du bactériophage. Les protéines considérées dans le cadre de la présente invention sont encadrées.

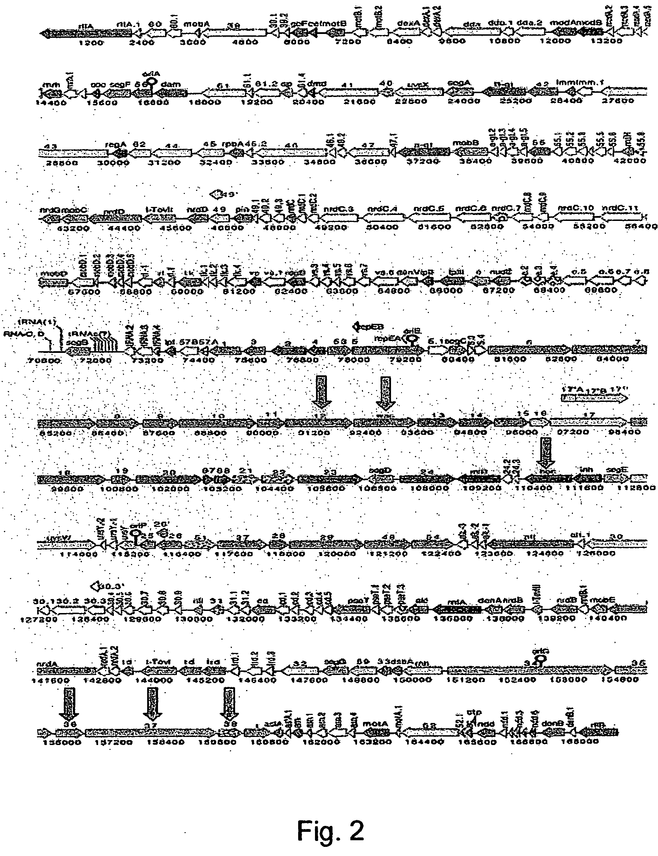

**Figure 2** : représentation du génome complet du bactériophage T4. Les gènes cités dans la présente demande sont indiqués à l'aide d'une flèche perpendiculaire aux cadres ouverts de lecture.
**Figure 3** : représentation en trois dimensions de la plate-forme basale du bactériophage et des fibres de queue impliquées dans la reconnaissance et l'adhésion du bactériophage à la bactérie hôte, permettant de visualiser les protéines de ciblage modifiées selon le procédé de l'invention.

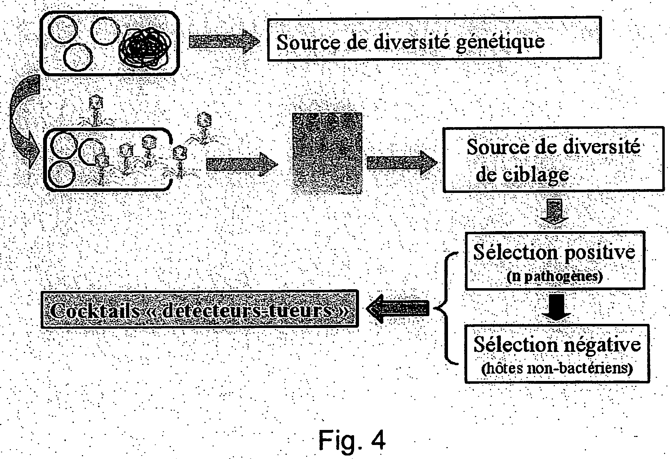

**Figure 4** : schéma résumant le principe d'obtention d'une banque de bactériophages recombinants selon l'invention. Le cadre en haut à gauche représente une bactérie hôte comprenant 3 vecteurs de recombinaison homologues visant à introduire des oligonucléotides dont la séquence est produite de manière aléatoire dans trois gènes codant les protéines de ciblage du bactériophage. Ces vecteurs représentent des constructions d'ADN au sens de la présente invention, porteuses d'une grande diversité génétique. Après infection par un bactériophage et recombinaison homologue, un grand nombre de bactériophages (banque de bactériophages) ayant des protéines de ciblage modifiées sont obtenus, formant « une source de diversité de ciblage ». Les bactériophages obtenus sont criblés vis-à-vis de nouveaux hôtes potentiels (sélection positive) afin de sélectionner des bactériophages capables d'infecter ces hôtes. Ils peuvent être également testés sur des hôtes non bactériens (cellules eucaryotes) pour s'assurer qu'ils ne sont pas dangereux pour l'homme ou les animaux.

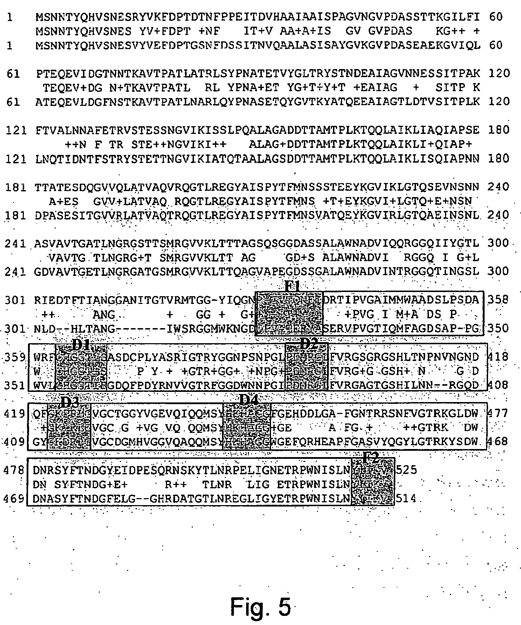

**Figure 5**: comparaison de la séquence polypeptidique de GP12 du bactériophage T4 (ligne haute) et de son homologue présente dans le bactériophage RB 69 (ligne basse) selon le protocole BLAST. Les acides aminés communs aux deux protéines sont indiqués sur la ligne intermédiaire. Le symbole « + » signifie que les acides aminés sont similaires. La partie N-terminale encadrée correspond au domaine de ces deux protéines qui permet l'ancrage du bactériophage sur la paroi de la bactérie. C'est ce domaine d'ancrage, qui est muté de manière aléatoire puis insérée par recombinaison homologue dans le génome du phage selon l'invention. Les segments internes D1 à D4 correspondent aux séquences de la protéine qui sont conservées au cours du procédé de diversification aléatoire mis en oeuvre par PCR selon l'invention.

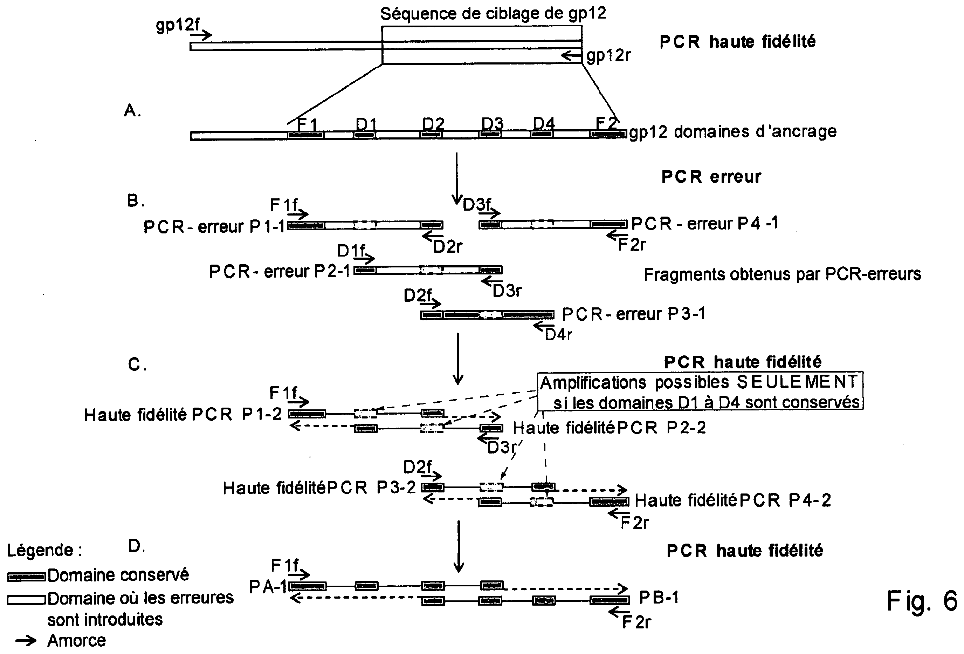

**Figure 6** : figure récapitulant les étapes du procédé par PCR mis en oeuvre selon l'invention pour obtenir, notamment, une copie du domaine d'ancrage de gp12 dans laquelle sont insérés des oligonucléotidiques produits de manière aléatoire. Les segments internes D1 à D4 dont on souhaite conserver l'identité de la séquence sont figurés par des rectangles. Ces domaines correspondent à ceux mentionnés dans la figure 5 ci-dessus. Les deux segments externes délimitent la séquence du domaine d'ancrage de gp12. **A** : le domaine d'ancrage de gp12 est amplifié par PCR haute fidélité. **B** : 4 PCR erreur sont réalisées de manière indépendante à l'aide des oligonucléotides mentionnés. **C** : Les produits d'amplifications obtenus en B sont purifiés et rassemblés dans une même réaction de PCR haute fidélité. Lesdits produits d'amplification se chevauchent si bien qu'il est possible d'assembler les différents fragments, mais à condition que les domaines D1 à D4 soient suffisamment conservés pour pouvoir hybrider les amorces utilisées. **D** : il résulte de la PCR réalisée à l'étape C, deux fragments PA-1 et PB-1 qui sont assemblés en utilisant les amorces correspondant aux segments externes du domaine d'ancrage de GP12. Au final, on obtient une copie du domaine d'ancrage de gp12 dont la séquence a été modifiée de manière aléatoire sauf au niveau des domaines D1 à D4 dont l'identité a été conservée. Les amorces utilisées dans l'exemple de réalisation ont été reportées à chacune des étapes mentionnées dans cette figure.

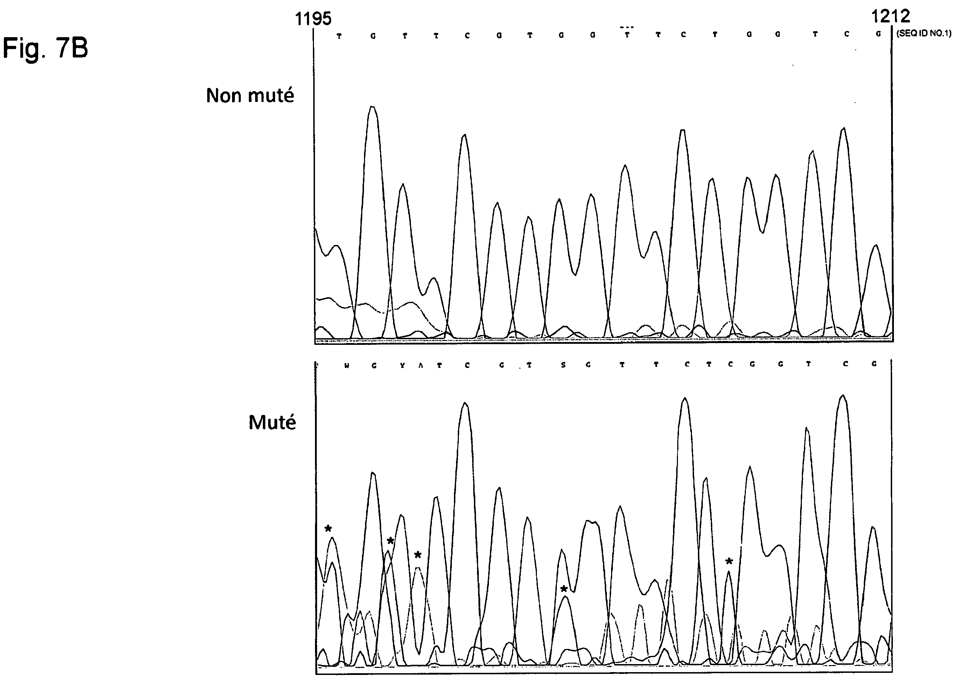

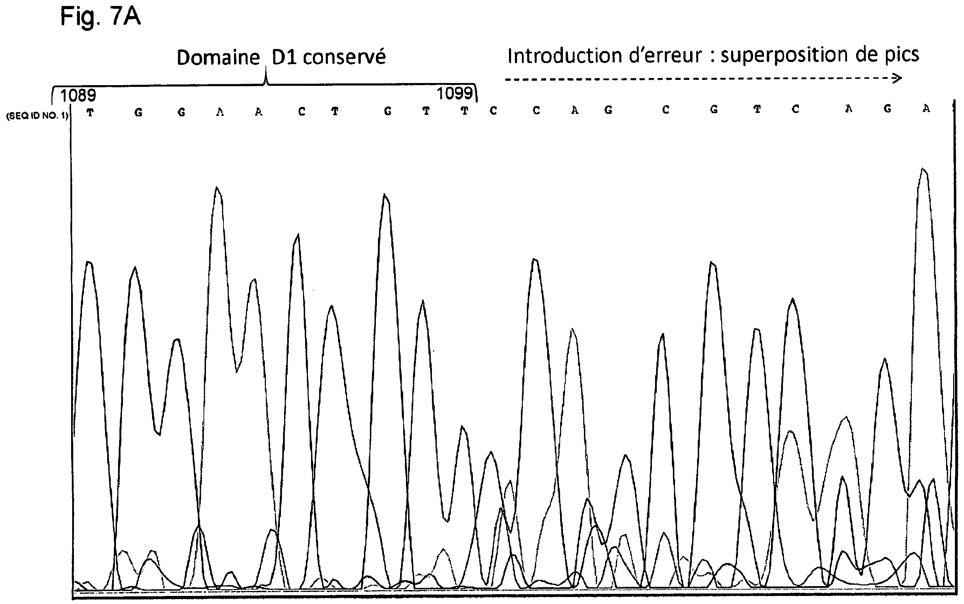

**Figure 7** : Profil de séquençage obtenu directement sur les produits de PCR (gp12-Mut) obtenus selon le procédé de l'invention. Les profils ont été établis en utilisant le logiciel d'analyse free ApE à partir des données provenant d'un séquenceur automatique. **A** : séquençage de la portion de gp12 situé entre les nucléotides 1089 et 1110 (SEQ ID NO.1). La partie gauche qui est conservée correspond à la partie 3' du domaine D1 (nucléotides 1089 à 1099). La partie droite montre une superposition de pics rendant compte des mutations aléatoires qui se sont produites au cours du procédé de PCR dans la région située immédiatement en aval de D1 (nucléotides 1100 à 1110). **B**: comparaison du séquençage réalisé dans une région non mutée de gp12 située entre les nucléotides 1195 et 1212 de SEQ ID NO.1 *(en haut)* et réalisé pour la même région sur les produits de PCR obtenus selon le procédé *(en bas).* La région séquencée se trouve entre les domaines conservés D1 et D2. On observe la présence de pics superposés *(en bas)* qui rendent compte de l'insertion aléatoire de nucléotides dans la séquence de *gp12* initiale.

### Description du procédé de diversification des séquences génétiques selon l'invention:

La présente invention a donc plus particulièrement pour objet un procédé de PCR permettant de muter de manière aléatoire une séquence nucléotidique S, délimitée à ses extrémités 5' et 3' par deux segments F1 et F2, en conservant l'identité d'au moins un segment interne D de ladite séquence nucléotidique, caractérisé en ce qu'il comprend les étapes suivantes:
i) on effectue une PCR-erreur sur la totalité de la séquence S à l'aide d'au moins deux amorces dont l'une est sens et l'autre antisens respectivement des segments F1 et F2, de sorte à amplifier la séquence S en y introduisant des mutations de manière aléatoire;
ii) on purifie les produits d'amplification obtenus, lesquels correspondent à des copies de S mutées de manière aléatoire;
iii) on effectue une PCR haute fidélité à partir des produits d'amplification purifiés à l'étape ii), en utilisant comme couples d'amorces sens et antisens respectivement au moins :
   - une amorce sens de F1 et une amorce antisens s'hybridant en totalité avec le segment D, afin d'amplifier la région F1-D des copies de S mutées,
   - une amorce sens s'hybridant en totalité avec le segment D et une amorce antisens de F2, afin d'amplifier la région D-F2 des copies de S mutées;
iv) on purifie les produits d'amplification obtenus à l'étape iii), lesquels consistent en des copies des segments F1-D et D-F2 de la séquence S mutée à l'étape i) dans lesquels le segment D a conservé son identité;
v) on effectue une PCR haute fidélité à partir des produits d'amplification purifiés à l'étape iv) en utilisant des amorces sens et antisens de respectivement F1 et F2;
vi) on purifie les produits de PCR obtenus, lesquels correspondent aux séquences nucléotidiques S mutées à l'étape i) dans lesquelles le segment D a conservé son identité.

Une amorce selon l'invention est un acide nucléique simple brin, apte à s'hybrider avec une portion, ou la totalité, de l'un des brins d'ADN formant tout ou partie de la séquence S. Une amorce est dite « sens » lorsque son enchainement de nucléotides reproduit, à quelques nucléotides prés, une partie du brin codant de S. Une amorce est dite « anti-sens » de S, lorsque son enchainement de nucléotides reproduit, à quelques nucléotides près, une partie du brin non codant de S.

Lorsqu'une amorce est donnée comme « correspondant à » un segment donné de la séquence S, cela signifie qu'elle peut être sens ou antisens par rapport à une partie de S pris dans sa forme de molécule d'ADN double brin.

Ce procédé est particulièrement avantageux lorsqu'on cherche à conserver l'identité de plusieurs domaines internes D_{N} de la séquence nucléotidique S (comme c'est le cas de la protéine GP12), N étant considéré comme un entier égal ou supérieur à 1.

Pour N=2, le procédé comprend alors les étapes suivantes:
i) on effectue une PCR-erreur sur la totalité de la séquence S à l'aide d'au moins deux amorces dont l'une est sens et l'autre antisens respectivement des segments F1 et F2, de sorte à amplifier la séquence S en y introduisant des mutations de manière aléatoire;
ii) on purifie les produits d'amplification obtenus;
iii) on effectue une PCR haute fidélité à partir des produits d'amplification purifiés à l'étape ii), en utilisant comme couples d'amorces sens et anti-sens au moins :
   - une amorce sens de F1 et une amorce anti-sens s'hybridant en totalité avec D₂, afin d'amplifier la région F1-D₂ des copies de S mutées dans lesquelles le segment D₂ a conservé son identité;
   - une amorce anti-sens de F2 et une amorce sens s'hybridant en totalité avec D₁, afin d'amplifier la région D₁-F2 de S mutées dans lesquelles le segment D₁ a conservé son identité;
iv) on effectue une PCR haute fidélité à partir des produits d'amplification obtenus à l'étape iii) en utilisant au moins un couple d'amorces sens et antisens de F1 et F2;
v) on purifie les produits de PCR obtenus à l'étape v), lesquels correspondent à des séquences nucléotidiques S mutées de manière aléatoire, dans lesquels les séquences des segments D₂ et D₁ ont conservé leur identité.

Selon un aspect préféré de l'invention ce procédé est utile pour introduire des oligonucléotides produits de manière aléatoire dans certains domaines variables des séquences codant les protéines de ciblage des bactériophages. Les protéines de ciblage du bactériophage se définissent comme des protéines qui participent à la reconnaissance et à l'adhésion du bactériophage à la bactérie hôte. Ces protéines sont de préférence choisies parmi celles constituant les fibres de queue ou de la plate-forme basale du phage T4. Une protéine particulièrement adaptée au procédé selon l'invention est la protéine GP12 de la plate forme basale dont la séquence nucléotidique correspond à SEQ ID N° 1. D'autres protéines de ciblage préférées sont GP36, GP37 ou GP38 constitutives de la partie distale des fibres de queue. Bien entendu, des protéines homologues de celles citées ci-dessus présentes chez d'autres bactériophages sont également préférées.

Par séquence homologue, il faut entendre des séquences ayant au moins 50 % d'identité avec ces dernières, de préférence au moins 70 %, plus préférentiellement au moins 90 % présente chez deux organismes d'espèces différentes.

L'exemple est donné dans la présente demande de la modification de la protéine GP12 du bactériophage T4, particulièrement préféré selon l'invention. La protéine GP12 présente un domaine d'ancrage dans lequel il est souhaitable d'introduire de la variabilité tout en maintenant l'intégrité de certains segments notés D1 à D4 (figure 5), lesquels segments correspondent à des domaines conservés chez les protéines homologues des bactériophages de type-T.

Préférentiellement, ce sont les segments des gènes des protéines correspondant aux domaines de la protéine impliqués dans les fonctions d'adhésion ou de reconnaissance qui sont modifiés, et plus préférentiellement les segments correspondent à des domaines variables.

Par domaine variable, il faut entendre les segments d'une séquence qui sont moins conservés lorsqu'on compare deux séquences homologues l'une à l'autre.

Inversement, il est préférable de conserver l'identité des domaines les plus conservés, lesquels sont généralement indispensables à la fonctionnalité des protéines codées, le cas échéant, par ces séquences.

Les protocoles de PCR utilisés selon l'invention sont des protocoles standards bien connus de l'homme du métier. C'est la manière dont ils sont mis en oeuvre qui fait la particularité de l'invention.

Par PCR-erreur, on entend une réaction de polymérisation en chaîne effectuée dans des conditions ne permettant pas une réplication fidèle des séquences d'ADN. Ce type de réaction peut être obtenu en mettant en oeuvre une polymérase classique de type *Taq* en condition faiblement stringente et en présence de sels de manganèse, ainsi que cela est décrit dans la littérature [Cadwell, R.C.et al. 1992, Randomization of gnes by PCR mutagenesis, PCR Methods Appl., 2:28-33].

Une PCR haute fidélité est, au contraire, une réaction de polymérisation en chaîne permettant d'amplifier une matrice d'ADN avec un taux d'erreur de réplication très faible. Ce type de réaction peut être obtenu en mettant en oeuvre, par exemple, une polymérase de type *Pfu* en condition stringente, ainsi que cela est décrit dans la littérature [Inmis, M.A. et al. Eds., PCR Protocols: a guide to methods and applications, 1989, Academic Press].

Les réactions de PCR haute fidélité mentionnées dans l'étape iii) qui permettent d'amplifier les sous-régions de la séquence S situées entre les segments conservés, sont de préférence effectuées de manière séparée.

Dans les exemples de la présente demande la lettre F désigne une amorce « sens » (en anglais forward) et la lettre R désigne une amorce anti-sens (en anglais reverse).

Le présent procédé peut s'appliquer à toute séquence nucléotidique dont on cherche à faire varier sélectivement et de manière aléatoire, certains segments.

Le procédé selon l'invention peut trouver des applications dans de nombreux domaines thérapeutiques.

Sa mise en oeuvre est, en effet particulièrement utile dès lors qu'on cherche à modifier le spectre d'activité, ou d'interaction avec un ligand, d'une protéine en modifiant aléatoirement des domaines particuliers de sa séquence génétique.

Ainsi, l'invention prévoit, par exemple, que la séquence S soit une séquence génétique codant une protéine d'intérêt.

Selon un aspect préféré de l'invention, la protéine d'intérêt est une protéine de ciblage d'un bactériophage, telle que la protéine GP12 d'un bactériophage de type T.

Cependant, il peut également s'agir de n'importe quelle protéine d'intérêt thérapeutique ou diagnostique dont on cherche à faire varier les sites actifs ou les domaines d'interaction, comme par exemple une protéine ligand dont on cherche à modifier la spécificité vis-à-vis d'un récepteur ou bien encore une immunoglobuline dont on cherche à modifier les domaines variables ou hypervariables dans le but de détecter des anticorps.

La présente demande ne saurait connaître de limitation du point de vue des nombreuses applications que laissent entrevoir la modification d'une séquence génétique selon l'invention.

Lorsque la séquence S est présente sous forme d'ARN, le présent procédé peut être précédé d'une étape de rétro-transcription permettant d'obtenir une copie de ladite séquence sous forme d'un polynucléotide selon les protocoles usuels.

Les produits d'amplifications obtenus selon le procédé particulier de l'invention constituent une banque de séquences nucléotidiques S diversifiées de manière aléatoire dont l'identité des domaines internes D_{N} a été conservée.

L'invention décrit donc des produits de PCR obtenus selon l'invention, lesquels sont caractérisés en ce qu'ils consistent en des variants polynucléotidiques résultant de la mutation aléatoire de la séquence S et caractérisés en ce qu'un ou plusieurs domaines internes D de ladite séquence S ont été conservés.

En d'autres termes, l'invention permet d'obtenir une banque de polynucléotides constituée de variants d'une séquence nucléotidique S mutée par PCR de manière aléatoire, caractérisée en ce que lesdits variants comprennent un ou plusieurs segment(s) interne(s) D de ladite séquence S intacts ou dont l'identité a été conservée.

En général les domaines internes D_{N} conservent, selon le procédé de l'invention, plus de 50 % d'identité par rapport à leur séquence initiale dans la protéine sauvage, de préférence plus de 70 %, et plus préférentiellement plus de 90 %, voire plus de 99 %, selon les conditions de PCR utilisées, notamment les conditions des PCR haute fidélité effectuées aux étapes iii) et v) du procédé selon l'invention.

Les variants polynucléotidiques constituant la banque de séquences nucléotidiques peuvent être directement clonées dans des vecteurs d'expression, ou plus préférentiellement dans des vecteurs de recombinaison homologue, dont l'homme du métier dispose, et former un ensemble de constructions selon l'invention.

Le procédé selon l'invention permet en particulier de surmonter la difficulté de muter par partie un gène de manière aléatoire. En effet, cela nécessitait dans l'art antérieur de produire des oligonucléotides de manière aléatoire puis d'assembler ces oligonucléotides par clonage aux parties de la séquence du gène dont on cherchait à conserver l'identité. Or, lorsque plusieurs oligonucléotides doivent être insérés à différents endroits du gène, ce travail est très fastidieux et le résultat en termes de diversité des séquences obtenues est décevant.

Les constructions d'ADN obtenues selon l'invention, prises dans leur ensemble, permettent ainsi plus particulièrement d'intégrer dans le génome un très grand nombre de séquences nucléotidiques différentes codant des protéines modifiées.

L'invention vise donc ainsi également un procédé de diversification d'une protéine codée par une séquence nucléotidique S, caractérisée en ce qu'on exprime dans un hôte d'expression approprié les variants polynucléotidiques de ladite séquence nucléotidique S contenu dans un produit de PCR ou une banque de polynucléotides tels que définis ci-dessus.

Ce procédé peut consister notamment en ce qu'on:
- on clone dans un vecteur d'expression les produits de PCR obtenus à l'étape vi) du procédé selon l'invention, lesquels correspondent aux séquences nucléotidiques S mutées dans lesquelles au moins un domaine D est resté identique, puis
- on transforme une cellule hôte permettant l'expression du polypeptide codée par la séquence S,
- on exprime dans ladite cellule hôte lesdites séquences nucléotidiques S mutées dans lesquelles au moins un domaine D est resté identique, pour obtenir des protéines diversifiées, et
- on purifie les différentes protéines obtenues.

Ces étapes sont effectuées par des techniques bien connues de l'homme du métier [Sambrook J., Russel D.W. (2001) Molecular Cloning, a Laboratory Manual, CHSL Press].

Une fois traduites en protéine, les séquences nucléotidiques contenues dans la banque susvisée peuvent résulter en l'expression de protéines présentant une diversité de séquences polypeptidiques produites de manière aléatoire incluant des domaines internes D_{N} dont l'identité de la séquence polypeptidique est conservée.

A cet égard l'invention décrit une banque de protéines résultant de l'expression d'une banque de séquences nucléotidique telle qu'indiquée ci-dessus, lesdites protéines présentant une diversité de séquences polypeptidiques produites de manière aléatoire incluant des segments internes D_{N} ayant conservé leur identité.

Le présent procédé permet donc la modification ciblée de toute protéine dont on désire changer certaines spécificités fonctionnelles, en faisant varier de manière aléatoire les séquences de ces protéines impliquées, par exemple, dans des interactions avec des ligands, des activités catalytiques, la toxicité ou le transport.

### Application des séquences diversifiées obtenues selon le procédé de l'invention à la transformation de bactériophages mutés dans leurs protéines de ciblage :

Selon un aspect préféré de l'invention, les produits d'amplifications obtenus sont insérés dans les gènes du bactériophage codant pour ses protéines de ciblage, au moyen de la recombinaison homologue.

Les constructions d'ADN préférées selon l'invention comprennent de préférence:
- une région permettant la duplication de ladite construction dans une bactérie hôte;
- une région permettant la recombinaison homologue dans le génome du bactériophage au niveau d'un gène codant une protéine de ciblage, ladite région comprenant deux séquences d'ADN homologues aux séquences dudit gène codant une protéine de ciblage, lesquelles délimitent un segment d'insertion incluant un oligonucléotide dont la séquence est produite de manière aléatoire, de préférence selon le procédé par PCR décrit ci-dessus.

Selon un aspect préféré de l'invention, la région permettant la recombinaison homologue comprend tout ou partie du gène codant la protéine de ciblage, de préférence la totalité de la séquence du gène.

De préférence, cette seconde région consiste en un produit d'amplification susceptible d'être obtenu selon le procédé de mutation aléatoire exposé ci-dessus.

Selon un aspect préféré de l'invention plusieurs gènes codant des protéines de ciblage du phage sont mutés simultanément par recombinaison homologue selon le procédé de l'invention. Pour parvenir à un tel résultat, l'invention prévoit de transformer la bactérie hôte successivement à l'aide de vecteurs différents, ciblant chacun un gène différent.

Les vecteurs préférés permettant de modifier simultanément les gènes GP12, GP37 ou GP38 du bactériophage T4 dans la bactérie hôte E.coli sont, par exemple, les vecteurs pACYC184 (ATCC 37033), pBAD18- K (ATCC 87397) et RR1 (ATCC 87076). De tels vecteurs présentent l'avantage de posséder des marqueurs conférant une résistance à des antibiotiques différents et ne partagent pas de séquences nucléotidiques communes susceptibles d'occasionner des recombinaisons entre les différents vecteurs une fois ceux-ci intégrés dans la bactérie hôte.

Un bactériophage est, de préférence, un bactériophage lytique, naturel ou modifié.

De préférence, le bactériophage utilisé est un phage de type-T, tels que les bactériophages T4, T5, T6 et T7, bien connus de l'homme du métier et plus particulièrement le phage T4, dont le génome a été séquencé [Miller, E.S. et al., Bacteriophage T4 genome, Microbiol Mol. Biol. Rev., 2003, 67(1):86-156]. La séquence complète du génome du bactériophage est disponible dans Genbank (AF 158101).

Une bactérie hôte est une bactérie utilisée couramment pour répliquer le phage que l'on cherche à modifier. De préférence, la bactérie hôte est une souche que l'on peut transformer à l'aide d'une construction d'ADN permettant de modifier le phage par recombinaison homologue.

Par transformation de cette bactérie hôte à l'aide des constructions d'ADN, on dispose d'une ou plusieurs banques de bactéries hôte transformées. Chacune des bactéries de cette banque contient potentiellement une construction capable de transformer par recombinaison homologue une ou plusieurs des protéines de ciblage du phage de manière différente.

Une telle banque de bactéries hôte présente l'avantage de pouvoir être multipliée et conservée. Elle constitue un produit intermédiaire renouvelable utile pour la production des bactériophages recombinants dont les protéines de ciblages sont modifiées de manière aléatoire.

Grâce au procédé selon invention, il est possible d'obtenir un ensemble de bactériophages recombinants très diversifié.

Ces bactériophages forment une banque de bactériophages au sens de la présente invention.

Pour qu'une banque de bactériophages recouvre le plus grand nombre possible de bactériophage différents, il convient de mettre en oeuvre un nombre suffisant de bactéries hôtes transformées, car c'est ce nombre qui détermine le nombre et la diversité des phages récoltés.

Si ce nombre est suffisant, une banque de bactériophages comprend au minimum, au moins 10⁶, de préférence 10⁸, plus préférentiellement 10¹⁰ variants différents d'un même bactériophage, lesdits variants se distinguant par la séquence d'au moins une de leurs protéines de ciblage.

La diversité des bactériophages dans la banque peut se justifier par un calcul simple de dénombrement.

Ainsi, si on considère que préférentiellement:
- 3 gènes codant des protéines de ciblages sont modifiés ; par l'insertion d'au moins 3 oligonucléotides composés de séquences aléatoires d'au moins 12 nucléotides; et que
- 1/3 des séquences nucléotidiques imposent une modification polypeptidique au niveau des protéines de ciblage ; et que
- seulement 3 des 4 bases (A, T, C, G) sont susceptibles de produire une mutation par rapport à la protéine initiale;

On obtient alors un minimum de 3²⁴ possibilités de mutations au niveau polypeptidique, ce qui revient à dénombrer quelques 2,8.10¹¹ bactériophages potentiellement différents.

Les protocoles expérimentaux qui suivent ont pour but d'illustrer l'invention à titre d'exemple sans limiter la portée de l'invention revendiquée.

### Préparation des séquences de variants de gp12, gp37 et gp38 de phages T4

Ce qui suit décrit le mode opératoire utilisé pour gp12, mais il peut être transposé sans difficulté pour l'homme du métier à la modification d'autres gènes codant notamment des protéines de ciblage.

### Etape 1 : Préparation du gène gp12

Le gène gp12 est amplifié par PCR à partir de l'ADN génomique du T4 de type sauvage obtenu à partir d'une culture concentrée de lysat de phages (voir ci-dessus) en utilisant comme amorces
gp12F 5'-TGAGTAATAATACATATCAACACG (SEQ ID No.2), et
gp12R 5'-TGATTCTTTTACCTTAATTATGTAC (SEQ ID No.3).

Après purification sur un gel d'agarose préparatif, le produit de PCR (gp12A) est utilisé comme matrice dans des réactions PCR sensibles à l'erreur avec pour but d'introduire des mutations et insertions ponctuelles dans la région codante correspondant au domaine de liaison au récepteur de gp12 (voir figure 5).

### Etape 2 : Introduction de mutations aléatoires dans le domaine de liaison au récepteur.

Une série de 4 réactions PCR emboîtées (40 cycles chacune) est mise en oeuvre en présence de Mn2 de manière à induire des erreurs de polymérase aléatoires.

Les PCR-erreur sont conduites dans un volume réactionnel de 100 µl, en utilisant des matrices et amorces à des concentrations finales respectivement égales à 400 ng et 30 pmol, avec 0,2 mM de dATP et dGTP (de chacun), 1 mM de dCTP et dTTP (de chacun), 2,5 mM de MgCl₂, 0,7 mM de MnCl₂ et 5 U d'ADN-polymérase *Taq* (New England Biolabs, Inc.) dans du tampon réactionnel 1×. La PCR est effectuée à 96 °C pendant 2 min, avec 30 cycles à 95 °C pendant 1 min, 56 °C pendant 1 min et 72 °C pendant 2 min, et une extension finale à 72 °C pendant 7 min.

La première réaction (P1-1) utilise comme amorces :
p12NF1F 5'-TCAAGGTAACCGCATCGTAAC (SEQ ID No.4)
p12NF2R 5'-AAAGACCACGCATGTCAG (SEQ ID No.5)

La deuxième réaction (P2-1) utilise comme amorces :
p12NF2F 5'-TGCCATGGTGGAACTGTTCA (SEQ ID No.6)
p12NF3R 5'-CACCTAATCTAGGTTTAC (SEQ ID No.7)

La troisième réaction (P3-1) utilise comme amorces :
p12NF3F 5'-CTGACATGCGTGGTCTTT (SEQ ID No.8)
p12NF4R 5'-ATGTTTATGATAAGACAT (SEQ ID No.9)

La quatrième réaction (P4-1) utilise comme amorces :
p12NF4F 5'-GTAAACCTAGATTAGGTG (SEQ ID No.10)
p12NF5R 5'-TCATTCTTTTACCTTAATTAT(SEQ ID No.11)

Chacun de ces produits de réaction présente un chevauchement partiel avec deux autres produits de réaction et les amorces utilisées correspondent à des domaines invariants conservés dans la protéine gp12. Ces domaines doivent être préservés fidèlement dans les structures de gènes mutés finales produites. Cependant, certains des fragments produits par les réactions PCR sensibles à l'erreur peuvent parfaitement avoir subi une mutagenèse dans ces régions. Afin de préserver des domaines intacts, chacun des produits précités doit alors être soumis à des réactions PCR à haute fidélité ayant pour but d'amplifier sélectivement seulement les fragments dans lesquels le domaine conservé a été retenu.

### Etape 3 : Amplification sélective des fragments désirés.

Celle-ci est effectuée par deux séries de réactions PCR à haute fidélité comprenant chacune 25 cycles.

Les PCR haute fidélité sont conduites dans un volume réactionnel de 50 µl, en utilisant des matrices et amorces à des concentrations finales respectivement égales à 250 ng et 40 pM, dans du tampon pour *pfu* 1× (Tris-HCl 20 mM à pH 9,0, KCI 10 mM, MgSO₄ 1 mM, (NH₄)₂SO₄ 6 mM, 0,1 % de Triton X-100, 0,1 mg/ml de SAB) avec 200 µM de dNTP et 5 U de polymérase *pfu* (Promega). Les profils de PCR sont les suivants : 94 °C pendant 20 s, 45 °C pendant 15 s et 72 °C pendant 30 s, avec répétition pendant 20 cycles.

Dans la première série de réactions (P1-2 à P4-2), une portion aliquote (environ 250 ng) de chacun des produits de PCR précités est amplifiée en utilisant les amorces «F» correspondantes (par exemple: p12NF1F) biotinylées à l'extrémité 5'. Cela est nécessaire pour séparer les produits désirés des matrices non amplifiables (domaines conservés mutés) qui ont des longueurs pratiquement identiques et qui ne peuvent être séparées par électrophorèse sur gel.

Les produits de chaque réaction sont ensuite passés sur des mini-colonnes d'exclusion dimensionnelle (pour éliminer l'excès d'amorces), purifiés individuellement sur des billes de streptavidine, lavés dans du tampon de liaison, élués, précipités et remis en suspension dans ddH₂O.

Dans la seconde série de réactions, une portion aliquote des produits de réaction P1-2 purifiés est mélangée à une quantité égale de produits de réaction P2-2. Ceux-ci ont en commun le site d'amorçage F2-R1 et F3-R2. En conséquence, le brin « - » des produits de réaction P2-2 sert d'amorce pour le brin « + » des produits de réaction P1-2 et l'extension avec une polymérase débute au site d'amorçage F2-R1 interne de P1-2. L'extension échoue pour les produits de réaction P1-2 où ce site conservé a été muté de manière significative.

De manière similaire, le brin « + » des produits de réaction P1-2 sert d'amorce pour le brin « - » des produits de réaction P2-2 et l'extension avec une polymérase débute au site d'amorçage F3-R2 interne de P2-2. L'extension échoue pour les produits de réaction P2-2 où ce site conservé a été muté de manière significative.

Les produits de PCR amplifiés avec succès (PA-1) correspondent à une fusion des fragments P1-2 et P2-2 dans laquelle chacun des quatre domaines conservés (F1 + F2-R1 + F3-R2 + F4-R3) a été retenu à l'état non muté.

Un mélange similaire est préparé à partir des produits de réaction P3-2 et P4-2. Dans ces réactions, une extension couronnée de succès est basée sur des sites conservés internes F4-R3 (P3-2) et F5-R4 (P4-2) intacts et les produits de PCR résultants (PA-1 et PB-1) correspondent à une fusion des fragments P3-2 et P4-2 dans laquelle chacun des quatre domaines conservés (F3-R2 + F4-R3 + F5-R4 + R5) a été obtenu à l'état non muté.

Les produits obtenus sont purifiés par électrophorèse sur gel préparatif puisque les dimensions des produits de PCR fusionnés sont très différentes de celles des matrices individuelles.

Afin d'augmenter le rendement dans cette seconde série de réactions PCR, le protocole d'extension peut être modifié de la manière suivante :
10 cycles avec seulement les produits P1-2 et P2-2, et P3-2 et P4-2 dans les mélanges réactionnels.

Les réactions sont interrompues, 50 ng de chacune des amorces p12NF1F et p12NF3R sont ajoutés au mélange PA-1 tandis que 50 ng de chacune des amorces p12NF3F et p12NF5R sont ajoutés au mélange PB-1, en laissant ensuite les réactions PCR se poursuivre pendant 15 cycles supplémentaires.

### Etape 4 : Reconstitution du domaine d'ancrage muté de gp12.

Après purification, des portions aliquotes égales des produits PA-1 et PB-1 sont utilisées ensemble dans une réaction PCR à haute fidélité comprenant 30 cycles ayant pour but de reconstituer le domaine total de liaison au récepteur de gp12 en fragments uniques renfermant les diverses mutations introduites ci-dessus.

Dans ce cas, comme dans la série précédente de réactions, les produits PA-1 servent d'amorces d'extension pour les produits PB-1 et *vice versa*.

Le produit de PCR fusionné final (gp12BD-Fu) est purifié par électrophorèse sur gel puisque ses dimensions sont très différentes de celles des matrices individuelles.

Afin d'augmenter le rendement, le protocole d'extension peut être modifié en procédant à une réaction comprenant 15 cycles avec seulement les produits P1-2 et P2-2, et P3-2 et P4-2 dans les mélanges réactionnels. Les réactions sont interrompues, 50 ng de chacune des amorces p12NF1 F et p12NF5R sont ajoutés au mélange, puis on laisse ensuite les réactions PCR se poursuivre pendant 15 cycles supplémentaires.

Après purification, une portion aliquote (environ 250 ng) du domaine de liaison au récepteur de gp12 reconstitué est utilisée comme matrice dans une nouvelle série de quatre réactions PCR sensibles à l'erreur, avec ensuite des amplifications sélectives et des reconstitutions des domaines de la manière décrite ci-dessus.

Cependant, puisque, à chaque étape de fusion (étapes 3 et 4 ci-dessus), toutes les mutations possibles introduites individuellement dans chaque sous-domaine sont brassées de manière aléatoire dans le domaine reconstitué final de liaison au récepteur, il existe des limites quant au nombre de cycles de mutagenèse qui peuvent être effectués sans effet négatif sur les domaines conservés. En raison des étapes nécessaires d'amplification sélective, il est estimé que, au-delà de 4 cycles successifs, toutes les mutations nouvellement introduites présenteront une forte probabilité d'effet négatif sur un domaine conservé ou d'introduction d'une réversion restituant la séquence T4 originale, non mutée.

### Etape 5 : Reconstitution de la copie modifiée du gène gp12 dans son entier

### 1) Amplification du segment de gp12 en amont du domaine de liaison au récepteur.

Le gène gp12, engendré par PCR, produit ci-dessus (gp12A) est utilisé comme matrice conjointement avec les amorces
gp12F 5'-TGAGTAATAATACATATCAACACG (SEQ ID No.12), et
gp12AR 5'-GTTACGATGCGGTTACCTTGT (SEQ ID No.13)
Il s'ensuit une purification des produits d'amplifications obtenus sur un gel d'agarose préparatif, une précipitation puis une remise en suspension dans ddH₂O.

### 2) Amplification du domaine de liaison muté de gp12

Une portion aliquote (environ 500 ng) du produit de PCR (gp12B) est utilisée conjointement avec une quantité égale de domaine de liaison au récepteur de gp12 reconstitué (gp12BD-Fu) dans une réaction PCR à haute fidélité. Il faut noter que ces deux produits présentent seulement en commun un chevauchement de 20 pb. En conséquence, le profil de la réaction PCR doit être modifié pour prendre ce fait en considération.

Profil de PCR modifié : A) 30 min à 96 °C puis 5 cycles d'1 min à 94 °C, de 10 s à 45 °C, et ensuite B) 5 cycles d'1 min à 94 °C et de 20 s à 50 °C, puis C) 5 cycles d' 1 min à 94 °C, de 30 s à 50 °C, et finalement D) 15 cycles d' 1 min à 94 °C, d'1 min à 55 °C, de 5 secondes à 72 °C.

Afin d'augmenter le rendement, la réaction peut être interrompue à la sous-étape D ci-dessus et les amorces gp12F et p12NF5R peuvent être introduites dans le mélange. On laisse ensuite la réaction se rétablir et s'effectuer jusqu'à son terme.

Les produits finals (gp12-Mut) sont purifiés sur un gel d'agarose préparatif, précipités et remis en suspension dans ddH₂O jusqu'à leur utilisation.

### Etape 6 : Vérification par séquençage des produits de PCR gp12-Mut obtenus

Les produits de PCR purifiés sur gel d'agarose sont séquencés à l'aide d'un séquenceur automatique afin de vérifier que des mutations ont bien été introduites de manière aléatoire dans gp12, tout en conservant intacte les domaines D1 et D2. Le séquençage est effectué sur deux portions correspondant respectivement aux nucléotides 1089 à 1110 (Fig. 6A) et 1195 et 1212 (Fig. 6B) de gp12 (SEQ ID NO.1). Les profils de séquençage obtenus sont présentés dans la Fig.6. En comparant les profils des produits de PCR à ceux de la séquence initiale de gp12, on peut constater que de nombreux pics supplémentaires viennent s'ajouter au profil attendu de gp12. Ces pics supplémentaires traduisent une fréquence élevée de mutations introduites dans la séquence par le procédé de PCR. Ces pics ne se retrouvent pas au niveau du domaine D1, ce qui confirme que ce domaine a conservé son identité de séquence avec celui de gp12.

### Clonage des gp12-Mut mutés dans un vecteur alternatif pour recombinaison homologue

Les modes opératoires précités ont permis d'engendrer, de façon similaire à gp12, les gènes mutants gp37 et gp38 de T4.

Le but des modes opératoires suivants est alors d'introduire ces gènes de T4 variants de manière à favoriser leur introduction, par recombinaison homologue, dans le génome du bactériophage T4 de manière à produire des descendances de T4 ayant des spécificités de ciblage d'hôtes différentes de celle du bactériophage parental.

A cet effet, les gènes mutés engendrés ci-dessus doivent être introduits dans des vecteurs alternatifs (un vecteur différent pour chaque gène). Dans le cas présent, les vecteurs pACYC184, pBAD18-K et RR1 sont utilisés pour cloner chacun des gènes mutés.

Le vecteur choisi (par exemple pACYC184 pour le clonage de gp12Mut) est coupé au niveau du site Sma I, purifié et remis en suspension dans 20 µl de ddH₂O et mélangé au produit de PCR gp12Mut en un rapport de 1:3. Après ligation, l'ADN est purifié, remis en suspension dans ddH₂O et transformé dans des cellules DK8 (ATCC 47038) « électrocompétentes ». Ce vecteur porte un gène de résistance au chloramphénicol (Chl). Ainsi, après une électroporation et une période de rétablissement de 1 h, les cellules sont transférées à 10 ml de milieu LB contenant 170 µg/ml de chloramphénicol et sont cultivées à 30 °C pendant 4 h avec aération constante. Puis les cellules sont étalées sur des plaques de milieu LB + Chl et cultivées pendant une nuit à 30 °C. Quelques colonies sont prélevées pour la vérification par PCR de la présence du segment d'insertion gp12Mut et quelques colonies positives sont cultivées dans du milieu LB + Chl pour la préparation d'une culture concentrée de cellules (DK8-p12C).

### Introduction des gènes mutés gp37 et gp38 dans la bactérie hôte

Une culture fraîche d'une nuit de DK8-p12C est utilisée pour préparer des cellules électrocompétentes.

Le vecteur pBAD18-K est coupé au niveau du site Sma I, purifié et remis en suspension dans 20 µl de ddH₂O et mélangé au produit de PCR gp37Mut en un rapport de 1:3. Après ligation, l'ADN est purifié, remis en suspension dans ddH₂O et transformé dans des cellules DK8-p12C « électrocompétentes ».

Le vecteur utilisé dans ce cas porte un gène de résistance à la kanamycine (Kan). Ainsi, après une électroporation et une période de rétablissement de 1 h, les cellules sont transférées à 10 ml de milieu LB contenant 170 µg/ml de chloramphénicol et 50 µg/ml de kanamycine et sont cultivées à 30 °C pendant 4 h avec aération constante. Puis les cellules sont étalées sur des plaques de milieu LB + Chl + Kan et cultivées pendant une nuit à 30 °C. Quelques colonies sont prélevées pour la vérification par PCR de la présence des segments d'insertion gp12Mut et gp37Mut et quelques colonies positives sont cultivées dans du milieu LB + Chl + Kan pour la préparation d'une culture concentrée de cellules (DK8-p12C-p37K).

Une culture fraîche d'une nuit de DK8-p12C-p37K est utilisée pour préparer des cellules électrocompétentes. Le vecteur RR1 est coupé au niveau du site Sma I, purifié et remis en suspension dans 20 µl de ddH₂O et mélangé au produit de PCR gp37Mut en un rapport de 1:3. Après ligation, l'ADN est purifié, remis en suspension dans ddH₂O et transformé dans des cellules DK8-p12C-p37K « électrocompétentes ». Le vecteur utilisé dans ce cas porte un gène de résistance à l'ampicilline (Amp). Ainsi, après une électroporation et une période de rétablissement de 1 h, les cellules sont transférées dans 10 ml de milieu LB contenant 170 µg/ml de chloramphénicol + 50 µg/ml de kanamycine + 60 µg/ml d'ampicilline et sont cultivées à 30 °C pendant 4 h avec aération constante. Puis les cellules sont étalées sur des plaques de milieu LB + Chl + Kan + Amp et cultivées pendant une nuit à 30 °C. Quelques colonies sont prélevées pour la vérification par PCR de la présence des segments d'insertion gp12Mut, gp37Mut et gp38Mut et quelques colonies positives sont cultivées dans du milieu LB + Chl + Kan + Amp pour la préparation d'une culture concentrée de cellules (DK8-p12C-p37K-P38A).

Il reste à présent à construire un hôte capable de présenter une puissance de recombinaison extrêmement efficace.

### Construction de bactérie hôtes E. coli « mini-λ »

Pour obtenir la recombinaison efficace de l'ADN donneur dans des fonds *recA*⁺ ou *recA*⁻, on prépare l'hôte *E. coli* qui contient un prophage λ portant les gènes de recombinaison exo, *bet* et *gam* sous le contrôle d'un répresseur cl de λ sensible à la température. Les gènes *exo*, *bet* et *gam* peuvent être aisément actionnés à 42 °C et inhibés à 32 °C. Lorsque les fonctions de λ sont actionnées pendant un temps réduit à 5 min, les cellules deviennent plus recombinogènes et absorbent l'ADN linéaire sans sa destruction. λ Gam inhibe l'attaque de l'ADN linéaire par la nucléase RecBCD de *E. coli*, et Exo et Beta engendrent une activité de recombinaison pour cet ADN linéaire. De manière plus importante, cette recombinaison est efficace avec des homologies d'ADN limitées à 30 à 50 pb aux extrémités des substrats consistant en ADN linéaire.

Les oligonucléotides 5' GTATGCATGCTGGGTGTGG (MλRf) et 5' CGCACTCTCGATTCGTAGAGCCTCG (MλRr) sont utilisés comme amorces pour l'amplification par PCR de l'ADN provenant de la région *attP-cro* en utilisant l'ADN de λ *cl857* comme matrice.

Une fois le prophage λ engendré par PCR, une possibilité consiste à le cloner le prophage λ dans le plasmide pFN476 (ATCC86962) à petit nombre de copies avec sélection par LacZ, par exemple.

Le vecteur pFN476 est coupé au niveau du site Sma I (extrémité franche), purifié, remis en suspension dans 20 µl de ddH₂O et mélangé au produit de PCR du prophage λ en un rapport de 1:3. Après ligation, l'ADN est purifié, remis en suspension dans ddH₂O et transformé dans des cellules DK8-p12C-p37K-p38A « électrocompétentes ». Après rétablissement, les cellules sont étalées sur des plaques de milieu LB X-gal + Chl + Kan + Amp (voir ci-dessus) et mises en incubation à 30 °C.

Quelques colonies blanches sont sélectionnées pour la vérification par PCR de la présence du prophage λ. Une colonie positive de prophages λ est ensuite cultivée pendant une nuit à 30 °C dans du milieu LB + Chl + Kan + Amp pour préparer une culture concentrée de cellules (DK8-T4Mut-λ) à utiliser dans les manipulations ultérieures.

A cette étape, les hôtes transformés sont inductibles par λ à température élevée, lacZ-positifs et contiennent des copies de gènes gp12, gp37 et gp38 de T4 mutés prêts pour la recombinaison homologue.

### Production de la descendance du bactériophage T4 avec des gammes d'hôtes étendues

Une culture fraîche d'une nuit de cellules DK8-T4Mut-λ est préparée dans du milieu LB + Chl + Kan + Amp à 30 °C.

Les cultures pour infection par T4 sont commencées avec un volume égal ou inférieur à 0,05 ml de cellules d'une culture d'une nuit pour 10 ml de milieu LB + Chl + Kan + Amp afin de garantir que les cellules passent à la phase de croissance exponentielle avant l'addition du bactériophage.

Pour améliorer l'aération, ces cultures sont multipliées dans des fioles d'Erlenmeyer de 250 ml à branche latérale, bouchées non hermétiquement, dans un bain d'eau d'agitateur par secousses à 30 °C.

250 ml de cellules dans du milieu LB + Chl + Kan + Amp sont cultivés à une densité de 3 × 10⁸ cellules par ml à 30 °C avec agitation par secousses.

Des portions aliquotes de 10 ml de cellules à croissance exponentielle sont ensuite transférées à 40 ml de milieu LB + Chl + Kan + Amp préchauffé à 42 °C et mises en incubation pendant exactement 15 min à 42 °C avec aération constante. Du tryptophane est ajouté à une concentration de 0,02 mg/ml et suivi par le bactériophage T4 à une multiplicité d'environ 10 particules par cellule. Les cultures sont transférées à un bain d'eau à 30 °C, en laissant la croissance se poursuivre pendant exactement 25 min.

Le but dans ce cas consiste à isoler la descendance de la première salve et à interrompre la propagation avant que cette descendance de bactériophages de première génération ait eu le temps de se transformer en reproducteurs.

### Récupération de la descendance des bactériophages

Les cellules sont recueillies par centrifugation à 5000 tr/min pendant 5 minutes et le surnageant est récupéré, quelques gouttes de chloroforme sont ajoutées et le mélange est centrifugé de nouveau pendant 10 min à 6000 tr/min. Le surnageant, en excluant le chloroforme, est ajusté à une concentration en tampon SM 1× (MgSO₄ 10 mM, NaCl 100 mM, 0,01 % de gélatine et Tris-HCl 50 mM [pH 7,5]) en utilisant du tampon concentré 5×, et est stocké à 4 °C avant l'analyse.

Les cellules rassemblées en un culot sont remises en suspension dans 8 ml de solution de Tris-chlorhydrate 0,05 M à pH 8,0 avec 25 % de saccharose. Une portion aliquote de 1,6 ml de lysozyme (5 mg/ml) est ajoutée et le mélange est mis en incubation pendant 5 min à 0 °C. Une portion aliquote de 3,2 ml de solution d'EDTA 0,2 M est ajoutée et le mélange est mis en incubation pendant une période de temps supplémentaire de 15 min à 0 °C. Le lysat cellulaire est ajusté à 500 mM de Tris-HCl à pH 7,4, 100 mM de MnCl₂ au moyen de tampon concentré 10×, mis à l'équilibre à 15 °C et mis en incubation avec 10 U de Dnase1 (Sigma) pendant 2 h. Le mélange est ensuite centrifugé à 6000 tr/min pendant 10 min. Le surnageant limpide est prélevé avec précaution, ajusté à une concentration en tampon SM 1× et stocké de la manière précitée.

### Vérification des extensions des gammes d'hôtes

Des cultures de souches bactériennes pathogènes (*Yersinia* sp., *Salmonella* sp., *E. coli* O157 H7, *Enterobacter sakazakii*, etc.) sont préparées.

Des portions aliquotes de 3 ml sont ensuite infectées avec 1 ml de culture concentrée de la descendance et mises en incubation à 30 °C avec agitation. La turbidité de la culture est déterminée par colorimétrie immédiatement après l'infection et ensuite toutes les 60 min. Une chute significative de la turbidité de culture en un temps de 5 heures indique que les particules capables d'infecter l'hôte testé étaient présentes dans la descendance de T4 recombinante. Quelques gouttes de chloroforme sont ajoutées à la ou les cultures, ce qui provoque une chute nette de turbidité, et les cultures sont ensuite centrifugées à 5000 tr/min pendant 5 min.

Le surnageant est récupéré et utilisé comme substance infectieuse pour la production de particules de bactériophages dédiées à la bactérie testée, laquelle dans la nature, ne peut être attaquée par le bactériophage sauvage de type T4.

## Revendications

1. Procédé de PCR permettant de muter de manière aléatoire une séquence nucléotidique S, délimitée à ses extrémités 5' et 3' par deux segments F1 et F2, tout en conservant l'identité d'un segment interne D de ladite séquence nucléotidique S, **caractérisé en ce qu'**il comprend les étapes suivantes :
i) on effectue une PCR-erreur sur la totalité de la séquence S à l'aide d'au moins deux amorces dont l'une est sens et l'autre antisens respectivement des segments F1 et F2, de sorte à amplifier la séquence S en y introduisant des mutations de manière aléatoire;
ii) on purifie les produits d'amplification obtenus, lesquels correspondent à des copies de S mutées de manière aléatoire;
iii) on effectue une PCR haute fidélité à partir des produits d'amplification purifiés à l'étape ii), en utilisant comme couples d'amorces sens et antisens respectivement au moins :
- une amorce sens de F1 et une amorce antisens s'hybridant en totalité avec le segment D, afin d'amplifier la région F1-D des copies de S mutées,
- une amorce sens s'hybridant en totalité avec le segment D et une amorce antisens de F2, afin d'amplifier la région D-F2 des copies de S mutées;
iv) on purifie les produits d'amplification obtenus à l'étape iii), lesquels consistent en des copies des segments F1-D et D-F2 de la séquence S mutée à l'étape i) dans lesquels le segment D a conservé son identité;
v) on effectue une PCR haute fidélité à partir des produits d'amplification purifiés à l'étape iv) en utilisant des amorces sens et antisens de respectivement F1 et F2;
vi) on purifie les produits de PCR obtenus, lesquels correspondent aux séquences nucléotidiques S mutées à l'étape i) dans lesquelles le segment D a conservé son identité.

2. Procédé pour diversifier par PCR une séquence nucléotidique S, délimitée à ses extrémités 5' et 3' par deux segments F1 et F2, tout en conservant l'identité de deux domaines internes D₁ et D₂ de ladite séquence nucléotidique S, **caractérisé en ce qu'**il comprend les étapes suivantes :
i) on effectue une PCR-erreur sur la totalité de la séquence S à l'aide d'au moins deux amorces dont l'une est sens et l'autre antisens respectivement des segments F1 et F2, de sorte à amplifier la séquence S en y introduisant des mutations de manière aléatoire;
ii) on purifie les produits d'amplification obtenus;
iii) on effectue une PCR haute fidélité à partir des produits d'amplification purifiés à l'étape ii), en utilisant comme couples d'amorces sens et anti-sens au moins :
- une amorce sens de F1 et une amorce anti-sens s'hybridant en totalité avec D₂, afin d'amplifier la région F1-D₂ des copies de S mutées dans lesquelles le segment D₂ a conservé son identité;
- une amorce anti-sens de F2 et une amorce sens s'hybridant en totalité avec D₁, afin d'amplifier la région D₁-F2 de S mutées dans lesquelles le segment D₁ a conservé son identité;
iv) on effectue une PCR haute fidélité à partir des produits d'amplification obtenus à l'étape iii) en utilisant au moins un couple d'amorces sens et antisens de F1 et F2;
v) on purifie les produits de PCR obtenus à l'étape v), lesquels correspondent à des séquences nucléotidiques S mutées de manière aléatoire, dans lesquels les séquences des segments D₂ et D₁ ont conservé leur identité.

3. Procédé selon la revendication 1 ou 2, **caractérisé en ce que** la séquence S est une séquence génétique codant une protéine d'intérêt.

4. Procédé selon la revendication 3, **caractérisé en ce que** la protéine d'intérêt est une protéine de ciblage d'un bactériophage, telle que la protéine GP12 d'un bactériophage de type T.

5. Procédé selon la revendication 3, **caractérisé en ce que** la protéine d'intérêt est une protéine ligand dont on cherche à modifier la spécificité vis-à-vis d'un récepteur.

6. Procédé selon la revendication 3, **caractérisé en ce que** la protéine d'intérêt est une immunoglobuline dont on cherche à modifier les domaines variables ou hypervariables.

## Patentansprüche

1. PCR-Verfahren, das eine Nukleotidsequenz S zufällig zu mutieren erlaubt, die an ihrem 5'- und 3'-Ende durch zwei Abschnitte F1 und F2 begrenzt ist, wobei die Identität eines internen Abschnitts D der Nukleotidsequenz S erhalten bleibt, **dadurch gekennzeichnet, dass** es die folgenden Schritte umfasst:
i) Durchführen einer fehlerhaften PCR in der gesamten S-Sequenz mit Hilfe von wenigstens zwei Primern, von denen einer ein Forward-Primer und der andere ein Reverse-Primer jeweils der Abschnitte F1 und F2 ist, so dass die S-Sequenz amplifiziert wird, wobei darin Mutationen zufällig eingeführt werden;
ii) Reinigen der erhaltenen Amplifikationsprodukte, die zufällig mutierten Kopien von S entsprechen;
iii) Durchführen einer sehr genauen PCR anhand der in Schritt ii) gereinigten Amplifikationsprodukte, wobei als Forward- und Reverse-Primer-Paare jeweils wenigstens verwendet werden:
- ein Forward-Primer von F1 und ein Reverse-Primer, der vollständig an dem Abschnitt D hybridisiert, um die Region F1-D der mutierten Kopien von S zu amplifizieren,
- ein Forward-Primer, der vollständig an dem Abschnitt D hybridisiert, und ein Reverse-Primer von F2, um die Region D-F2 der mutierten Kopien von S zu amplifizieren;
iv) Reinigen der in Schritt iii) erhaltenen Amplifikationsprodukte, die aus Kopien der Abschnitte F1-D und D-F2 der in Schritt i) mutierten S-Sequenz bestehen, in denen der Abschnitt D seine Identität beibehalten hat;
v) Durchführen einer sehr genauen PCR anhand der in Schritt iv) gereinigten Amplifikationsprodukte, wobei Forward- und Reverse-Primer jeweils von F1 und F2 verwendet werden;
vi) Reinigen der erhaltenen PCR-Produkte, die den in Schritt i) mutierten Nukleotidsequenzen S entsprechen, in denen der Abschnitt D seine Identität beibehalten hat.

2. Verfahren zum Diversifizieren einer Nukleotidsequenz S durch PCR, die an ihrem 5'- und 3'-Ende durch zwei Abschnitte F1 und F2 begrenzt ist, wobei die Identität von zwei internen Bereichen D₁ und D₂ der Nukleotidsequenz S erhalten bleibt, **dadurch gekennzeichnet, dass** es die folgenden Schritte umfasst:
i) Durchführen einer fehlerhaften PCR in der gesamten S-Sequenz mit Hilfe von wenigstens zwei Primern, von denen einer ein Forward-Primer und der andere ein Reverse-Primer jeweils der Abschnitte F1 und F2 ist, so dass die S-Sequenz amplifiziert wird, wobei darin Mutationen zufällig eingeführt werden;
ii) Reinigen der erhaltenen Amplifikationsprodukte;
iii) Durchführen einer sehr genauen PCR anhand der in Schritt ii) gereinigten Amplifikationsprodukte, wobei als Forward- und Reverse-Primer-Paare wenigstens verwendet werden:
- ein Forward-Primer von F1 und ein Reverse-Primer, der vollständig an D₂ hybridisiert, um die Region F1-D₂ der mutierten Kopien von S zu amplifizieren, in denen der Abschnitt D₂ seine Identität beibehalten hat;
- ein Reverse-Primer von F2 und ein Forward-Primer, der vollständig an D₁ hybridisiert, um die Region D₁-F2 der mutierten Kopien von S zu amplifizieren, in denen der Abschnitt D₁ seine Identität beibehalten hat;
iv) Durchführen einer sehr genauen PCR anhand der in Schritt iii) erhaltenen Amplifikationsprodukte, wobei wenigstens ein Forward- und Reverse-Primer-Paar von F1 und F2 verwendet wird;
v) Reinigen der in Schritt iv) erhaltenen PCR-Produkte, die den zufällig mutierten Nukleotidsequenzen S entsprechen, in denen die Abschnitte D₂ und D₁ ihre Identität beibehalten haben.

3. Verfahren nach Anspruch 1 oder 2,
**dadurch gekennzeichnet, dass** die S-Sequenz eine Gensequenz ist, die ein interessierendes Protein codiert.

4. Verfahren nach Anspruch 3,
**dadurch gekennzeichnet, dass** das interessierende Protein ein zielführendes Protein eines Bakteriophagen ist, wie etwa das Protein GP12 eines T-Phagen.

5. Verfahren nach Anspruch 3,
**dadurch gekennzeichnet, dass** das interessierende Protein ein Ligandprotein ist, dessen Spezifität gegenüber einem Rezeptor verändert werden soll.

6. Verfahren nach Anspruch 3,
**dadurch gekennzeichnet, dass** das interessierende Protein ein Immunglobulin ist, dessen variable oder hypervariable Bereiche verändert werden sollen.

## Claims

1. Method of PCR permitting random mutation of a nucleotide sequence S, delimited at its 5' and 3' ends by two segments F1 and F2, while preserving the identity of an inner segment D of said nucleotide sequence S, **characterized in that** it comprises the following steps:
i) an error-prone PCR is performed on the whole of sequence S using at least two primers, one of which is sense and the other antisense respectively of segments F1 and F2, so as to amplify sequence S, introducing mutations into it randomly;
ii) the amplification products obtained, which correspond to randomly mutated copies of S, are purified;
iii) high-fidelity PCR is performed starting from the amplification products purified in step ii), using as sense and antisense primer pairs, respectively, at least:
- a sense primer of F1 and an antisense primer hybridizing with the whole of segment D, in order to amplify region F1-D of the mutated copies of S,
- a sense primer hybridizing with the whole of segment D and an antisense primer of F2, in order to amplify region D-F2 of the mutated copies of S,
iv) the amplification products obtained in step iii), which consist of copies of segments F1-D and D-F2 of sequence S mutated in step i), in which segment D has preserved its identity, are purified;
v) high-fidelity PCR is performed starting from the amplification products purified in step iv) using sense and antisense primers of respectively F1 and F2;
vi) the PCR products obtained, which correspond to the nucleotide sequences S mutated in step i), in which segment D has preserved its identity, are purified.

2. Method for diversifying, by PCR, a nucleotide sequence S, delimited at its 5' and 3' ends by two segments F1 and F2, while preserving the identity of two inner domains D₁ and D₂ of said nucleotide sequence S, **characterized in that** it comprises the following steps:
i) an error-prone PCR is performed on the whole of sequence S using at least two primers of which one is sense and the other antisense respectively of segments F1 and F2, so as to amplify sequence S, introducing mutations into it randomly;
ii) the amplification products obtained are purified;
iii) high-fidelity PCR is performed starting from the amplification products purified in step ii), using as sense and antisense primer pairs at least:
- a sense primer of F1 and an antisense primer hybridizing with the whole of D₂, in order to amplify region F1-D₂ of the mutated copies of S in which segment D₂ has preserved its identity;
- an antisense primer of F2 and a sense primer hybridizing with the whole of D₁, in order to amplify mutated region D₁-F2 of S in which segment D₁ has preserved its identity;
iv) high-fidelity PCR is performed starting from the amplification products obtained in step iii) using at least one pair of sense and antisense primers of F1 and F2;
v) the PCR products obtained in step iv), which correspond to randomly mutated nucleotide sequences S, in which the sequences of the segments D₂ and D1 have preserved their identity, are purified.

3. Method according to claim 1 or 2, **characterized in that** sequence S is a genetic sequence encoding a protein of interest.

4. Method according to claim 3, **characterized in that** the protein of interest is a targeting protein of a bacteriophage, such as protein GP12 of a type T bacteriophage.

5. Method according to claim 3, **characterized in that** the protein of interest is a ligand protein for which it is sought to modify the specificity with respect to a receptor.

6. Method according to claim 3, **characterized in that** the protein of interest is an immunoglobulin for which it is sought to modify the variable or hypervariable domains.


### LISTAGE DE SEQUENCES

<110> PHERECYDES PHARMA
<120> Procédé de diversification aléatoire d'une séquence génétique permettant de préserver l'identité de certains segments internes de ladite séquence génétique
<130> BIF 117147 WO
<160> 13
<170> PatentIn version 3.1
<210> 1
   <211> 1582
   <212> DNA
   <213> bacteriophage T4
<220>
   <221> misc_feature
   <222> (1) .. (1582)
   <223> Région codante du gène gp12 de bactériophage T4
<400> 1
   gagtaataat acatatcaac acgtttctaa tgaatctcgt tatgtaaaat ttgatcctac 60
   cgatacgaat tttccaccgg agattactga tgttcacgct gctatagcag ccatttctcc 120
   tgctggagta aatggagttc ctgatgcatc gtcaacaaca aagggaattc tatttattcc 180
   cactgaacag gaagttatag atggaactaa taataccaaa gcagttacac cagcaacgtt 240
   ggcaacaaga ttatcttatc caaatgcaac tgaaactgtt tacggattaa caagatattc 300
   aaccaatgat gaagccattg ccggagttaa taatgaatct tctataactc cagctaaatt 360
   tactgtcgcc cttaataatg cgtttgaaac gcgagtttca actgaatcct caaatggtgt 420
   tattaaaatt tcatctctac cgcaagcatt agctggtgca gatgatacta ctgcaatgac 480
   tccattaaaa acacagcagt tagctattaa attaattgcg caaattgctc cttctgaaac 540
   cacagctacc gaatcggacc aaggtgttgt tcaattagca acagtagcgc aggttcgtca 600
   gggaacttta agagaaggct atgcaatttc tccttatacg tttatgaatt catcttctac 660
   tgaagaatat aaaggcgtaa ttaaattagg aacacaatca gaagttaact cgaataatgc 720
   ttctgttgcg gttactggcg caactcttaa tggtcgtggt tctacgacgt caatgagagg 780
   cgtagttaaa ttaactacaa ccgccggttc acagagtgga ggcgatgctt catcagcctt 840
   agcttggaat gctgacgtta tccagcaaag aggtggtcaa attatctatg gaacactccg 900
   cattgaagac acatttacaa tagctaatgg tggagcaaat attacgggta ccgtcagaat 960
   gactggcggt tatattcaag gtaaccgcat cgtaacacaa aatgaaattg atagaactat 1020
   tcctgtcgga gctattatga tgtgggccgc tgatagtctt cctagtgatg cttggcgctt 1080
   ctgccatggt ggaactgttt cagcgtcaga ttgtccatta tatgcttcta gaattggaac 1140
   aagatatggc ggaaacccat caaatcctgg attgcctgac atgcgtggtc tttttgttcg 1200
   tggttctggt cgtggttctc acttaacaaa tccaaatgtt aatggtaatg accaatttgg 1260
   taaacctaga ttaggtgtag gttgtaccgg tggatatgtt ggtgaagtac agatacaaca 1320
   gatgtcttat cataaacatg ctggtggatt tggtgagcat gatgatctgg gggcattcgg 1380
   taatacccgt agatcaaatt ttgttggtac acgtaaagga cttgactggg ataaccgttc 1440
   atacttcacc aatgacggat atgaaattga cccagaatca caacgaaatt ccaaatatac 1500
   attaaatcgt cctgaattaa ttggaaatga aacacgtcca tggaacattt ctttaaacta 1560
   cataattaag gtaaaagaat ga 1582
<210> 2
   <211> 24
   <212> DNA
   <213> Séquence artificielle
<220>
   <221> Amorce
   <222> (1) .. (24)
   <223> Amorce gp12F pour l'amplification du gène gp12 du bacteriophage T4
<400> 2
   tgagtaataa tacatatcaa cacg 24
<210> 3
   <211> 25
   <212> DNA
   <213> Séquence artificielle
<220>
   <221> Amorce
   <222> (1)..(25)
   <223> Amorce gp12R pour l'amplification du gène gp12 du bacteriophage T4
<400> 3
   tgattctttt accttaatta tgtac 25
<210> 4
   <211> 21
   <212> DNA
   <213> Séquence artificielle
<220>
   <221> Amorce
   <222> (1)..(21)
   <223> Amorce P12NF1F interne du domaine d'ancrage de gp12 du
   bacteriophage T4
<400> 4
   tcaaggtaac cgcatcgtaa c 21
<210> 5
   <211> 18
   <212> DNA
   <213> Séquence artificielle
<220>
   <221> Amorce
   <222> (1)..(18)
   <223> Amorce P12NF2R interne du domaine d'ancrage de gp12 du
   bacteriophage T4
<400> 5
   aaagaccacg catgtcag 18
<210> 6
   <211> 20
   <212> DNA
   <213> Séquence artificielle
<220>
   <221> Amorce
   <222> (1)..(20)
   <223> Amorce P12NF2F interne du domaine d'ancrage de gp12 du
   bacteriophage T4
<400> 6
   tgccatggtg gaactgttca 20
<210> 7
   <211> 18
   <212> DNA
   <213> Séquence artificielle
<220>
   <221> Amorce
   <222> (1)..(18)
   <223> Amorce P12NF3R interne du domaine d'ancrage de gp12 du
   bacteriophage T4
<400> 7
   cacctaatct acgtttac 18
<210> 8
   <211> 18
   <212> DNA
   <213> Séquence artificielle
<220>
   <221> Amorce
   <222> (1)..(18)
   <223> Amorce P12NF3F interne du domaine d'ancrage de gp12 du
   bacteriophage T4
<400> 8
   ctgacatgcg tggtcttt 18
<210> 9
   <211> 18
   <212> DNA
   <213> Séquence artificielle
<220>
   <221> Amorce
   <222> (1)..(18)
   <223> Amorce P12NF4R interne du domaine d'ancrage de gp12 du
   bacteriophage T4
<400> 9
   atgtttatga taagacat 18
<210> 10
   <211> 18
   <212> DNA
   <213> Séquence artificielle
<220>
   <221> Amorce
   <222> (1)..(18)
   <223> Amorce P12NF4F interne du domaine d'ancrage de gp12 du
   bacteriophage T4
<400> 10
   gtaaacctag attaggtg 18
<210> 11
   <211> 21
   <212> DNA
   <213> Séquence artificielle
<220>
   <221> Amorce
   <222> (1) .. (21)
   <223> Amorce P12NF5R interne du domaine d'ancrage de gp12 du
   bacteriophage T4
<400> 11
   tcattctttt accttaatta t 21
<210> 12
   <211> 24
   <212> DNA
   <213> Séquence artificielle
<220>
   <221> Amorce
   <222> (1) .. (24)
   <223> Amorce gp12F pour l'amplification du gène gp12 du bacteriophage T4
<400> 12
   tgagtaataa tacatatcaa cacg 24
<210> 13
   <211> 21
   <212> DNA
   <213> Séquence artificielle
<220>
   <221> Amorce
   <222> (1)..(21)
   <223> Amorce gp12AR pour l'amplification du gène gp12 du bacteriophage T4
<400> 13
   gttacgatgc ggttaccttg t 21